# EUROPEAN PATENT SPECIFICATION

(11) **EP 3 366 666 B1**
(45) Date of publication and mention of the grant of the patent: **18.11.2020**
(21) Application number: 16856546.3
(22) Date of filing: 11.01.2016
(51) Int. Cl.: C07C 209/60, C07C 209/68, C07C 211/54

(54) **PREPARATION METHOD FOR ARYL-SUBSTITUTED P-PHENYLENEDIAMINE SUBSTANCE**
VERFAHREN ZUR HERSTELLUNG EINER ARYLSUBSTITUIERTEN P-PHENYLENDIAMINSUBSTANZ
PROCÉDÉ DE PRÉPARATION D'UNE SUBSTANCE P-PHÉNYLÈNEDIAMINE À SUBSTITUTION ARYLE

(30) Priority: 21.10.2015 CN 201510691388
(43) Date of publication of application: 29.08.2018
(73) Proprietor: Sennics Co., Ltd., Pudong New Area Shanghai 200126 (CN)
(72) Inventor: GUO, Xiangyun, Shanghai 200126 (CN); XING, Jinguo, Shanghai 200126 (CN); RUAN, Xiaomin, Shanghai 200126 (CN); CHEN, Xinmin, Shanghai 200126 (CN)
(74) Representative: Biggi, Cristina
(86) International application number: PCT/CN2016/070614
(87) International publication number: WO 2017/067098

(56) References cited:
- EP-A1- 0 103 990
- CN-A- 101 489 991
- CN-A- 102 584 596
- US-A- 4 804 783
- US-A- 4 804 783
- WANG, WEI ET AL.: 'New Synthesis of Phenyl/Phenyl End-Capped Tetraaniline in the Leucoemeraldine and Emeraldine Oxidation States' SYNTHETIC METALS vol. 129, no. 2, 10 July 2002, ISSN 0379-6779 pages 199 - 205, XP055377514

## Description

### Technical Field

The present disclosure relates to the technical field of organic synthesis, and particularly to a preparation method for an aryl substituted p-phenylenediamine compounds.

### Background

An aryl substituted p-phenylenediamine compound is an important one in p-phenylenediamine derivatives, and is a compound or mixture (called as a mixture because it may comprise different compounds and these compounds have the same parent nucleus structure but different substituted aryls), which is prepared by performing aryl substitution on an N-amino hydrogen atom and N'-amino hydrogen atom in p-phenylenediamine. A rubber aging inhibitor 3100 is an important one in aryl substituted p-phenylenediamine compound.

The rubber aging inhibitor 3100, with a chemical name of N,N'-dimethylphenyl p-phenylenediamine or diaryl p-phenylenediamine, includes three compounds with different structures, and structural formulae of the three compounds are as follows respectively:

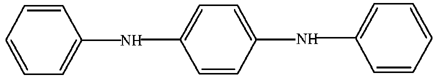

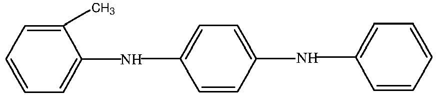

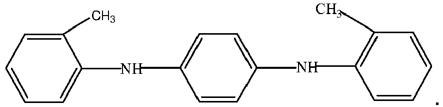

The rubber aging inhibitor 3100 is a typical delayed action type p-phenylenediamine rubber aging inhibitor. It may effectively overcome the shortcoming for present dominant p-phenylenediamine aging inhibitors 4020 and 4010NA, which have good early aging inhibition effects but slightly poor post aging inhibition effects. The rubber aging inhibitor 3100 is applied to synthetic rubber such as natural rubber, cis-butadiene rubber, styrene-butadiene rubber, nitrile butadiene rubber and chloroprene rubber, and belongs to a variety of efficient aging inhibitors with extremely good ozone-resistant protection effects for tires.

At present, a main production process for a product is as follows: p-dihydroxybenzene, aniline and o-toluidine are taken as reaction raw materials, and are reacted in a normal-pressure or high-pressure kettle in the presence of a lewis acid (for example, anhydrous ferric chloride) catalyst. In this process, toluene is adopted as a water entraining solvent to continuously entrain water produced by reaction out of a reaction system to promote the reaction to be performed towards a direction of producing the product. A reaction temperature reaches about 250°C, and an amount of the entrained water is used as a mark for determining that the reaction is ended. After the reaction is ended, the temperature is reduced, a saturated sodium carbonate aqueous solution is added for quenching reaction. Then the temperature is increased to remove a low-boiling-point substance by reduced-pressure distillation, an inorganic solvent is filtered when it is hot, and an organic phase is washed for many times to obtain the product. The advantage of the process is that the whole process flow is relatively simple. The shortcomings are that the adopted reaction raw material p-dihydroxybenzene is expensive, limited in supply channel and relatively unstable in cost; alkali liquor is required to be used to quench the lewis acid and wash away metal ions catalyst remaining in the product, so that a great amount of wastewater is produced; and equipment is greatly corroded by the high reaction temperature and the acid medium. In EP0103990, an improved process for preparing diarylamines from alicyclic ketones, a primary aromatic amine, and a hydrogen acceptor, in the presence of a platinum metal catalyst and acid promoter. EP0103990 describes a process for preparing diarylamines from alicyclic ketones, a primary aromatic amine, and a hydrogen acceptor, in the presence of a platinum metal catalyst and acid promoter. The process described by this document is carried out in the presence of an acid promoter and the reaction mechanism provides that, in the presence of a catalyst, an alicyclic ketone (such as cyclohexanone), a primary aromatic amine and a hydrogen acceptor react together, and that the latter is converted into the primary aromatic amine with a molar ratio of ketone to primary aromatic amine in the range of 0.68:1 to 1.65:1.

Like the rubber aging inhibitor 3100, present synthesis processes for aryl substituted p-phenylenediamine compound all have problems of high cost, poor environment friendliness, high corrosiveness and strict reaction condition. Therefore, it is necessary to provide a green preparation process for preparing an aryl substituted p-phenylenediamine compound, which is with the characteristics of low cost, high environment friendliness, mild reaction condition, relatively low corrosiveness to reaction equipment and the like.

### Summary

A main purpose of the disclosure is to provide a preparation method for one or more aryl substituted p-phenylenediamine compounds, so as to solve the problems of high cost, poor environment friendliness, high corrosiveness and strict reaction condition when the aryl substituted p-phenylenediamine substance is synthesized in a conventional art.

In order to achieve the purpose, according to an aspect of the disclosure, a preparation method for one or more aryl substituted p-phenylenediamine compounds is provided, a structural formula of the aryl substituted p-phenylenediamine compounds being as follows: 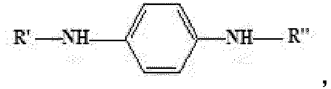 where R" and R' are chosen independently from phenyl or o-methylphenyl; and
the preparation method comprises that: reacting a raw material A and a raw material B and a hydrogen acceptor in the presence of a catalyst to form the aryl substituted p-phenylenediamine compounds, the raw material A having a structure shown as Formula I wherein R' is chosen from phenyl or o-methylphenyl, thus being N-phenyl p-phenylenediamine or N-o-methylphenyl p-phenylenediamine, the raw material B being cyclohexanone and/or o-methylcyclohexanone and the hydrogen acceptor being phenol and/or o-cresol which can accept hydrogen for conversion into the raw material B, a molar ratio of the raw material A and the raw material B is 20:1∼5:1, and a molar ratio of the raw material A and the hydrogen acceptor is 1:10∼1:2.5

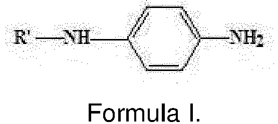

Furthermore, the aryl substituted p-phenylenediamine compounds are a mixture of N,N'-diphenyl p-phenylenediamine, N-phenyl-N'-methylphenyl p-phenylenediamine and N,N'-di(methylphenyl) p-phenylenediamine, and the preparation method comprises the following steps: taking N-phenyl p-phenylenediamine as the raw material A, taking the cyclohexanone and/or the o-methylcyclohexanone as the raw material B, taking the phenol as the hydrogen acceptor, and performing reaction in the presence of the catalyst to obtain a first component; taking the N-phenyl p-phenylenediamine as the raw material A, taking the cyclohexanone and/or the o-methylcyclohexanone as the raw material B, taking the o-cresol as the hydrogen acceptor, and performing reaction in the presence of the catalyst to obtain a second component, or, taking N-o-methylphenyl p-phenylenediamine as the raw material A, taking the cyclohexanone and/or the o-methylcyclohexanone as the raw material B, taking the phenol as the hydrogen acceptor, and performing reaction in the presence of the catalyst to obtain a second component; taking the N-o-methylphenyl p-phenylenediamine as the raw material A, taking the cyclohexanone and/or the o-methylcyclohexanone as the raw material B, taking the o-cresol as the hydrogen acceptor, and performing reaction in the presence of the catalyst to obtain a third component; and mixing the first component, the second component and the third component to obtain the mixture of N,N'-diphenyl p-phenylenediamine, N-phenyl-N'-methylphenyl p-phenylenediamine and N,N'-di(methylphenyl) p-phenylenediamine.

Furthermore, the aryl substituted p-phenylenediamine compounds are a mixture of N,N'-diphenyl p-phenylenediamine, N-phenyl-N'-methylphenyl p-phenylenediamine and N,N'-di(methylphenyl) p-phenylenediamine, and the preparation method comprises the following steps: taking a mixture of the N-phenyl p-phenylenediamine and the N-o-methylphenyl p-phenylenediamine as the raw material A, taking the cyclohexanone and/or the o-methylcyclohexanone as the raw material B, taking a mixture of the phenol and the o-cresol as the hydrogen acceptor, and performing reaction in the presence of the catalyst to obtain the mixture of N,N'-diphenyl p-phenylenediamine, N-phenyl-N'-methylphenyl p-phenylenediamine and N,N'-di(methylphenyl) p-phenylenediamine.

Furthermore, the catalyst is a supported noble metal catalyst, and is preferably a Pd-C and/or Pt-C supported noble metal catalyst.

Furthermore, a using amount of the catalyst is 0.1∼2% of a weight of the raw material A.

Furthermore, the raw material A and the raw material B, and the hydrogen acceptor are reacted for reaction time of 6∼8h under a temperature condition of 220∼280°C.

Furthermore, after the reaction is finished, the preparation method further comprises: filtering reaction liquid obtained by the reaction is to obtain filtrate, and performing reduced-pressure distillation on the filtrate to obtain the aryl substituted p-phenylenediamine compound.

Furthermore, the raw material A and the raw material B and the hydrogen acceptor are reacted in a nitrogen atmosphere.

With application of the technical solution of the disclosure, the raw material A with the structure shown as Formula I is reacted with the cyclohexanone and/or the o-methylcyclohexanone and a hydrogen acceptor to generate the aryl substituted p-phenylenediamine compounds. Specifically, the cyclohexanone and the o-methylcyclohexanone both may undergo dehydrogenation reaction in the presence of the catalyst, and meanwhile, a hydrogen atom on amino at an N' position in the raw material A is substituted to form the aryl substituted p-phenylenediamine compounds of which the R" is the phenyl or the o-methylphenyl. In addition, in the presence of the catalyst, the hydrogen acceptor in a reaction system continuously accepts hydrogen released from the cyclohexanone and/or the o-methylcyclohexanone to further form the raw material B to supply the cyclohexanone and/or o-methylcyclohexanone required by the reaction. In the preparation method, addition of the raw material B may be very small, and it is mainly taken as a primer raw material for the reaction. The raw material B may provide the required hydrogen for the hydrogen acceptor when being reacted with the raw material A in the presence of the catalyst, and a large amount of aryl substituted p-phenylenediamine compounds are formed in processes of continuous hydrogen accepting of the hydrogen acceptor and continuous dehydrogenation of the generated compound B. According to the preparation method, the raw materials are low in cost and readily available, and a large amount of water is avoided to use for posttreatment of the reaction. Moreover, a reaction condition is relatively mild, and corrosion to reaction equipment is avoided. Therefore, the preparation method is environment-friendly and less in pollution, and may achieve better economic benefits.

### Detailed Description of the Embodiments

The disclosure will be described below in combination with the embodiments in detail.

As described in the Background, existing synthesis processes for aryl substituted p-phenylenediamine compounds all have the problems of high cost, poor environment friendliness, high corrosiveness and complex process. In order to solve these problems, the disclosure provides a preparation method for one or more aryl substituted p-phenylenediamine compounds, a structural formula of the aryl substituted p-phenylenediamine compounds being as follows: 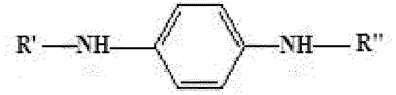 where R" and R' are chosen independently from phenyl or o-methylphenyl; and
the preparation method comprises that: reacting a raw material A and a raw material B and a hydrogen acceptor in the presence of a catalyst to form the aryl substituted p-phenylenediamine compounds, the raw material A having a structure shown as Formula I wherein R' is chosen from phenyl or o-methylphenyl, thus being N-phenyl p-phenylenediamine or N-o-methylphenyl p-phenylenediamine, the raw material B being cyclohexanone and/or o-methylcyclohexanone and the hydrogen acceptor being phenol and/or o-cresol which can accept hydrogen for conversion into the raw material B, , a molar ratio of the raw material A and the raw material B is 20:1∼5:1, and a molar ratio of the raw material A and the hydrogen acceptor is 1:10∼1:2.5

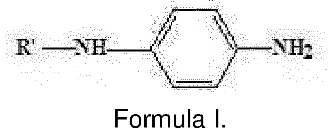

According to the preparation method provided by the disclosure, the raw material A with the structure shown as Formula I is reacted with the raw material B (cyclohexanone and/or o-methylcyclohexanone) and a hydrogen acceptor to generate the aryl substituted p-phenylenediamine compounds. Specifically, the cyclohexanone and the o-methylcyclohexanone both may undergo dehydrogenation reaction in the presence of the catalyst, and meanwhile, a hydrogen atom on amino at an N' position in the raw material A is substituted to form the aryl substituted p-phenylenediamine compounds of which R" is the phenyl or the o-methylphenyl. In addition, the hydrogen acceptor in a reaction system continuously accepts hydrogen released from the cyclohexanone and/or the o-methylcyclohexanone in the presence of the catalyst to further form the raw material B to supply the cyclohexanone and/or o-methylcyclohexanone required by the reaction. In the preparation method, addition of the raw material B may be very small, it is mainly taken as a primer raw material for the reaction, and may provide the required hydrogen for the hydrogen acceptor when being reacted with the raw material in the presence of the catalyst, and a large amount of aryl substituted p-phenylenediamine compounds are formed in processes of continuous hydrogen accepting of the hydrogen acceptor and continuous dehydrogenation of the generated compound B.

According to the preparation method provided by the disclosure, the raw materials are low in cost and readily available, and use of a large amount of water for posttreatment of the reaction is avoided. Moreover, a reaction condition is relatively mild, and corrosion to reaction equipment is avoided. Therefore, the preparation method is environment-friendly and less in pollution, and may achieve better economic benefits. Controlling a relationship between using amounts of each raw material within the abovementioned range may effectively ensure a reaction rate, ensure sufficient hydrogen in the reaction system and continuously convert the hydrogen acceptor into the raw material B for further reaction with the raw material A. Meanwhile, the raw material B which is relatively high in cost may further be saved, so that the preparation method is endowed with higher economy. The phenol and the o-cresol have higher conversion efficiency when accepting hydrogen for conversion into the cyclohexanone and the o-methylcyclohexanone, and meanwhile, the two hydrogen acceptors are relatively low in cost and more suitable to be used for industrial production in large doses.

It is important to note that the aryl substituted p-phenylenediamine compound may be a compound, and may also be a mixture. When the adopted raw material A is a single structured compound and the raw material B is the cyclohexanone or the o-methylcyclohexanone, the prepared aryl substituted p-phenylenediamine compounds are also a single structured compound. When the adopted raw material A and/or raw material B are/or mixtures/a mixture of multiple compounds, the reaction may still be performed, and the obtained aryl substituted p-phenylenediamine compounds are a mixture formed by two or more than two compounds with the same parent nucleus structure but different substituted aryl. Specifically, a skilled in the art may select different raw materials to be matched to obtain different aryl substituted p-phenylenediamine compounds, which will not be elaborated herein.

As long as the preparation method provided by the disclosure is adopted, the aryl substituted p-phenylenediamine compounds with a specific structure may be prepared. In a preferred implementation mode, the aryl substituted p-phenylenediamine compounds are a mixture of N,N'-diphenyl p-phenylenediamine, N-phenyl-N'-methylphenyl p-phenylenediamine and N,N'-di(methylphenyl) p-phenylenediamine, and the preparation method comprises the following steps: taking N-phenyl p-phenylenediamine as the raw material A, taking the cyclohexanone and/or the o-methylcyclohexanone as the raw material B, taking the phenol as the hydrogen acceptor, and performing reaction in the presence of the catalyst to obtain a first component (as mentioned above, the addition of the raw material B may be very small, it may provide the required hydrogen for the hydrogen acceptor when being reacted with the raw material A in the presence of the catalyst, and a large amount of aryl substituted p-phenylenediamine compounds are formed in the processes of continuous hydrogen accepting of the hydrogen acceptor and continuous dehydrogenation of the generated compound B. In this process, although the cyclohexanone and/or the o-methylcyclohexanone are/is taken as the raw material B, since the addition is relatively small, a main component of a final product is still the first component); taking the N-phenyl p-phenylenediamine as the raw material A, taking the cyclohexanone and/or the o-methylcyclohexanone as the raw material B, taking the o-cresol as the hydrogen acceptor, and performing reaction in the presence of the catalyst to obtain a second component, or, taking N-o-methylphenyl p-phenylenediamine as the raw material A, taking the cyclohexanone and/or the o-methylcyclohexanone as the raw material B, taking the phenol as the hydrogen acceptor, and performing reaction in the presence of the catalyst to obtain a second component (similarly, in this process, although the cyclohexanone and/or the o-methylcyclohexanone are/is taken as the raw material B, since the addition is relatively small, a main component of a final product is still the second component); taking the N-o-methylphenyl p-phenylenediamine as the raw material A, taking the cyclohexanone and/or the o-methylcyclohexanone as the raw material B, taking the o-cresol as the hydrogen acceptor, and performing reaction in the presence of the catalyst to obtain a third component (similarly, although the cyclohexanone and/or the o-methylcyclohexanone are/is taken as the raw material B, a main component of product generated by the step is still the third component); and mixing the first component, the second component and the third component to obtain the rubber aging inhibitor 3100.

There exists no sequence between the preparation steps of the three components in the preparation method. As a skilled in the art know, the rubber aging inhibitor 3100 is a mixture of three aryl substituted p-phenylenediamine compounds, and actually, in the preparation steps, the single raw material A, raw material B and hydrogen acceptor are adopted to prepare the three components and the three components are finally mixed to obtain the rubber aging inhibitor 3100. In a mixing process, a skilled in the art are only required to set respective using amounts of the three components according to a predetermined requirement of the rubber aging inhibitor 3100.

Of course, besides the abovementioned sub-steps for preparing the rubber aging inhibitor 3100, a one-step method may also be adopted to prepare the rubber aging inhibitor 3100, and the preparation method comprises the following steps: taking a mixture of the N-phenyl p-phenylenediamine and the N-o-methylphenyl p-phenylenediamine as the raw material A, taking the cyclohexanone and/or the o-methylcyclohexanone as the raw material B, taking a mixture of the phenol and the o-cresol as the hydrogen acceptor, and performing reaction in the presence of the catalyst to obtain the mixture of N,N'-diphenyl p-phenylenediamine, N-phenyl-N'-methylphenyl p-phenylenediamine and N,N'-di(methylphenyl) p-phenylenediamine, as the rubber aging inhibitor 3100. Preparing the rubber aging inhibitor 3100 with such an one-step method may further simplify reaction procedures and reduce reaction cost.

In the preparation method provided by the disclosure, the adopted catalyst is only required to have dehydrogenation and hydrogenation functions. In a preferred implementation mode, the catalyst is a supported noble metal catalyst, and is preferably a Pd-C and/or Pt-C supported noble metal catalyst. These catalysts have relatively high catalytic activity, and may make the reaction condition milder. In addition, a skilled in the art may regulate a specific using amount of the catalyst. In a preferred implementation mode, a using amount of the catalyst is 0.1∼2% of a weight of the raw material A.

As mentioned above, due to a unique reaction principle and route in the disclosure, the synthesis condition for the aryl substituted p-phenylenediamine compounds are milder. In a preferred implementation mode, the raw material A and the raw material B and the hydrogen acceptor are reacted for reaction time of 6∼8h under a temperature condition of 220∼280°C. Controlling the temperature and time of the reaction system within the abovementioned ranges may increase a conversion rate and a speed and is also favorable for reducing a side reaction rate of the reaction and endowing the product with higher purity.

More preferably, after the reaction is finished, the preparation method further comprising: filtering reaction liquid obtained by the reaction to obtain filtrate, and performing reduced-pressure distillation on the filtrate to obtain the aryl substituted p-phenylenediamine compound. Due to the unique reaction route during the reaction, a posttreatment process is relatively simple. Furthermore, the raw material A and the raw material B and the hydrogen acceptor are preferably reacted in a nitrogen atmosphere.

The application will further be described below in combination with specific embodiments in detail, and these embodiments should not be understood to limit the scope of protection of the application.

### Embodiment 1

36.8g (0.2mol) of N-phenyl p-phenylenediamine, 94.1g (1mol) of phenol, 2.0g (0.02mol) of cyclohexanone and 1.3g of dry Pd/C (containing 5wt% Pd) catalyst are added into a 500mL autoclave; and stirring is started, heating is performed to increase a temperature to 220°C after nitrogen displacement is performed for 3 times, heat is preserved to perform reaction for 6h, the temperature is reduced to a room temperature for discharging, filtering is performed, the catalyst is recovered, reduced-pressure distillation is performed on filtrate to remove unreacted phenol and cyclohexanone to obtain 51.7g of distillation residual liquid, sampling is performed to measure the content of N,N'-diphenyl p-phenylenediamine to be 94.1%, and the yield is calculated to be 93.6%.

### Embodiments 2 to 5

The same raw materials and process conditions in embodiment 1 are adopted to prepare N,N'-diphenyl p-phenylenediamine, and differences are differences in relationships between using amounts of each raw material (wherein a weight of the N-phenyl p-phenylenediamine is kept unchanged). Specific relationships between the using amounts and product conditions are as follows:

| | Molar ratio of N-phenyl p-phenylenedi amine and cyclohexanone | Molar ratio of N-phenyl p-phenylenediami ne and phenol | Weight percent of the catalyst in N-phenyl p-phenylenedi amine | Product content (%) | Yield (%) |
|---|---|---|---|---|---|
| Embodi ment 2 | 5:1 | 1:2.5 | 0.1% | 67.5 | 75.1 |
| Embodi ment 3 | 10:1 | 1:5 | 0.5% | 68 | 75.1 |
| Embodi ment 4 | 15:1 | 1:7.5 | 1% | 68.3 | 79.6 |
| Embodi ment 5 | 20:1 | 1:10 | 2% | 80.4 | 89.7 |

### Embodiment 6

36.8 g (0.2mol) of N-phenyl p-phenylenediamine, 108.1g (1mol) of o-cresol, 2.0g (0.02mol) of cyclohexanone and 1.3g of dry Pt/C (containing 5wt% Pt) catalyst are added into a 500mL autoclave; and stirring is started, heating is performed to increase a temperature to 250°C after nitrogen displacement is performed for 3 times, heat is preserved to perform reaction for 6h, the temperature is reduced to a room temperature for discharging, filtering is performed, the catalyst is recovered, reduced-pressure distillation is performed on filtrate to remove unreacted o-cresol and cyclohexanone to obtain 49.7g of distillation residual liquid, sampling is performed to measure the content of N-phenyl-N'-methylphenyl p-phenylenediamine to be 68%, and the yield is calculated to be 90.8%.

### Embodiment 7

39.6g (0.2mol) of N-methylphenyl p-phenylenediamine, 94.1g (1mol) of phenol, 2.0g (0.02mol) of cyclohexanone and 1.3g of dry Pd/C (containing 5wt% Pd) catalyst are added into a 500mL autoclave; and stirring is started, heating is performed to increase a temperature to 250°C after nitrogen displacement is performed for 3 times, heat is preserved to perform reaction for 6h, the temperature is reduced to a room temperature for discharging, filtering is performed, the catalyst is recovered, reduced-pressure distillation is performed on filtrate to remove unreacted phenol and cyclohexanone to obtain 53.7g of distillation residual liquid, sampling is performed to measure the content of N-phenyl-N'-methylphenyl p-phenylenediamine to be 93.8%, and the yield is calculated to be 91.9%.

### Embodiment 8

39.6g (0.2mol) of N-methylphenyl p-phenylenediamine, 108.1g (1mol) of o-cresol, 2.0g (0.02mol) of cyclohexanone and 1.3g of dry Pd/C (containing 5wt% Pd) catalyst are added into a 500mL autoclave; and stirring is started, heating is performed to increase a temperature to 270°C after nitrogen displacement is performed for 3 times, heat is preserved to perform reaction for 6h, the temperature is reduced to a room temperature for discharging, filtering is performed, the catalyst is recovered, reduced-pressure distillation is performed on filtrate to remove unreacted o-cresol and cyclohexanone to obtain 56.6g of distillation residual liquid, sampling is performed to measure the content of N,N'-di(methylphenyl) p-phenylenediamine to be 93.4%, and the yield is calculated to be 91.8%.

### Embodiment 9

18.4g (0.1mol) of N-phenyl p-phenylenediamine, 19.8g (0.1mol) of N-methylphenyl p-phenylenediamine, 94.1g (1mol) of phenol, 2.0g (0.02mol) of cyclohexanone and 1.3g of dry Pd/C (containing 5wt% Pd) catalyst are added into a 500mL autoclave; and stirring is started, heating is performed to increase a temperature to 250°C after nitrogen displacement is performed for 3 times, heat is preserved to perform reaction for 6h, the temperature is reduced to a room temperature for discharging, filtering is performed, the catalyst is recovered, reduced-pressure distillation is performed on filtrate to remove unreacted phenol and cyclohexanone to obtain 52g of distillation residual liquid, sampling is performed to measure contents of N,N'-diphenyl p-phenylenediamine and N-phenyl-N'-methylphenyl p-phenylenediamine to be 47.2% and 46.8% respectively, and yields are calculated to be 94.4% and 88.8% respectively.

### Embodiment 10

18.4g (0.1mol) of N-phenyl p-phenylenediamine, 19.8g (0.1mol) of N-methylphenyl p-phenylenediamine, 108.1g (1mol) of o-cresol, 2.0g (0.02mol) of cyclohexanone and 1.3g of dry Pd/C (containing 5wt% Pd) catalyst are added into a 500mL autoclave; and stirring is started, heating is performed to increase a temperature to 270°C after nitrogen displacement is performed for 3 times, heat is preserved to perform reaction for 8h, the temperature is reduced to a room temperature for discharging, filtering is performed, the catalyst is recovered, reduced-pressure distillation is performed on filtrate to remove unreacted phenol and cyclohexanone to obtain 54.2g of distillation residual liquid, sampling is performed to measure contents of N-phenyl-N'-methylphenyl p-phenylenediamine and N,N'-di(methylphenyl) p-phenylenediamine and to be 47.3% and 47.2% respectively, and yields are calculated to be 93.6% and 88.8% respectively.

### Embodiment 11

18.4g (0.1mol) of N-phenyl p-phenylenediamine, 19.8g (0.1mol) of N-methylphenyl p-phenylenediamine, 47.1g (0.5mol) of phenol, 54.1g (0.5mol) of o-cresol, 2.0g (0.02mol) of cyclohexanone and 1.3g of dry Pd/C (containing 5wt% Pd) catalyst are added into a 500mL autoclave; and stirring is started, heating is performed to increase a temperature to 270 °C after nitrogen displacement is performed for 3 times, heat is preserved to perform reaction for 8h, the temperature is reduced to a room temperature for discharging, filtering is performed, the catalyst is recovered, reduced-pressure distillation is performed on filtrate to remove unreacted phenol and cyclohexanone to obtain 53.4g of distillation residual liquid, sampling is performed to measure contents of N,N'-diphenyl p-phenylenediamine, N-phenyl-N'-methylphenyl p-phenylenediamine and N,N'-di(methylphenyl) p-phenylenediamine to sequentially be 42.2%, 37.5% and 14.1%, and this product may directly be used as a rubber aging inhibitor 3100.

### Embodiment 12

27.6g (0.15mol) of N-phenyl p-phenylenediamine, 9.9g (0.05mol) of N-methylphenyl p-phenylenediamine, 23.5g (0.25mol) of phenol, 81.1g (0.75mol) of o-cresol, 2.0g (0.02mol) of cyclohexanone and 1.3g of dry Pd/C (containing 5wt% Pd) catalyst are added into a 500mL autoclave; and stirring is started, heating is performed to increase a temperature to 280 °C after nitrogen displacement is performed for 3 times, heat is preserved to perform reaction for 8h, the temperature is reduced to a room temperature for discharging, filtering is performed, the catalyst is recovered, reduced-pressure distillation is performed on filtrate to remove unreacted phenol and cyclohexanone to obtain 54.5g of distillation residual liquid, sampling is performed to measure contents of N,N'-diphenyl p-phenylenediamine, N-phenyl-N'-methylphenyl p-phenylenediamine and N,N'-di(methylphenyl) p-phenylenediamine to sequentially be 22.6%, 43.7% and 24.2%, and this product may directly be used as a rubber aging inhibitor 3100.

From the above descriptions, it can be seen that the embodiments of the disclosure achieve the following technical effects.

According to the preparation method, the raw materials are low in cost and readily available, and use of a large amount of water for posttreatment of the reaction is avoided. Moreover, the reaction condition is relatively mild, and corrosion to reaction equipment is avoided. Therefore, the preparation method is environment-friendly and less in pollution, and may achieve better economic benefits. More particularly, controlling a proportion of each raw material and the reaction process condition may further increase the product content and yield of the product.

## Claims

1. A preparation method for one or more aryl substituted p-phenylenediamine compounds as defined in a structural formula as follows: 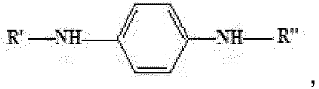 Where R" and R' are chosen independently from phenyl or o-methylphenyl; and
the preparation method comprises: reacting a raw material A, a raw material B, and a hydrogen acceptor in the presence of a catalyst to form the aryl substituted p-phenylenediamine compounds, the raw material A having a structure shown as Formula I wherein R' is chosen from phenyl or o-methylphenyl, thus being N-phenyl p-phenylenediamine or N-o-methylphenyl p-phenylenediamine, the raw material B being cyclohexanone and/or o-methylcyclohexanone and the hydrogen acceptor being phenol and/or o-cresol which can accept hydrogen for conversion into the raw material B, 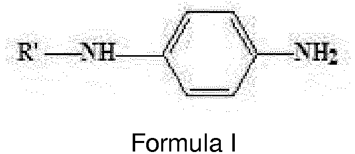 wherein a molar ratio of the raw material A and the raw material B is 20:1∼5:1 and a molar ratio of the raw material A and the hydrogen acceptor is 1:10∼1:2.5.

2. The preparation method as claimed in claim 1, wherein the aryl substituted p-phenylenediamine compounds are a mixture of N,N'-diphenyl p-phenylenediamine, N-phenyl-N'-methylphenyl p-phenylenediamine and N,N'-di(methylphenyl) p-phenylenediamine, and the preparation method comprises the following steps:
taking N-phenyl p-phenylenediamine as the raw material A, taking the cyclohexanone and/or the o-methylcyclohexanone as the raw material B, taking the phenol as the hydrogen acceptor, and performing reaction in the presence of the catalyst to obtain a first component;
taking the N-phenyl p-phenylenediamine as the raw material A, taking the cyclohexanone and/or the o-methylcyclohexanone as the raw material B, taking the o-cresol as the hydrogen acceptor, and performing reaction in the presence of the catalyst to obtain a second component, or, taking N-o-methylphenyl p-phenylenediamine as the raw material A, taking the cyclohexanone and/or the o-methylcyclohexanone as the raw material B, taking the phenol as the hydrogen acceptor, and performing reaction in the presence of the catalyst to obtain a second component;
taking the N-o-methylphenyl p-phenylenediamine as the raw material A, taking the cyclohexanone and/or the o-methylcyclohexanone as the raw material B, taking the o-cresol as the hydrogen acceptor, and performing reaction in the presence of the catalyst to obtain a third component; and
mixing the first component, the second component and the third component to obtain the mixture of N,N'-diphenyl p-phenylenediamine, N-phenyl-N'-methylphenyl p-phenylenediamine and N,N'-di(methylphenyl) p-phenylenediamine.

3. The preparation method as claimed in claim 1, wherein the aryl substituted p-phenylenediamine compounds are a mixture of N,N'-diphenyl p-phenylenediamine, N-phenyl-N'-methylphenyl p-phenylenediamine and N,N'-di(methylphenyl) p-phenylenediamine, and the preparation method comprises the following steps:
taking a mixture of the N-phenyl p-phenylenediamine and the N-o-methylphenyl p-phenylenediamine as the raw material A, taking the cyclohexanone and/or the o-methylcyclohexanone as the raw material B, taking a mixture of the phenol and the o-cresol as the hydrogen acceptor, and performing reaction in the presence of the catalyst to obtain the mixture of N,N'-diphenyl p-phenylenediamine, N-phenyl-N'-methylphenyl p-phenylenediamine and N,N'-di(methylphenyl) p-phenylenediamine.

4. The preparation method as claimed in any one of claims 1-3, wherein the catalyst is a supported noble metal catalyst, and is preferably the catalyst is a Pd-C and/or Pt-C supported noble metal catalyst.

5. The preparation method as claimed in claim 4, wherein a using amount of the catalyst is 0.1∼2% by weight of the raw material A.

6. The preparation method as claimed in any one of claims 1-3, wherein the raw material A and the raw material B, and the hydrogen acceptor are reacted for a reaction time of 6∼8h under a temperature condition of 220∼280 °C.

7. The preparation method as claimed in claim 6, wherein after the reaction is finished, the preparation method further comprising: filtering reaction liquid obtained by the reaction to obtain filtrate, and performing reduced-pressure distillation on the filtrate to obtain the aryl substituted p-phenylenediamine compound.

8. The preparation method as claimed in claim 6, wherein the raw material A, the raw material B and the hydrogen acceptor are reacted in a nitrogen atmosphere.

## Patentansprüche

1. Verfahren zur Herstellung für eine oder mehrere arylsubstituierte p-Phenylendiaminverbindungen, wie in einer Strukturformel wie folgt definiert: 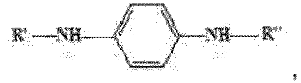 wobei R" und R' unabhängig von Phenyl oder o-Methylphenyl ausgewählt werden; und
das Verfahren zur Herstellung umfasst: Umsetzen eines Rohmaterials A, eines Rohmaterials B und eines Wasserstoffakzeptors in Gegenwart eines Katalysators zur Bildung der arylsubstituierten p-Phenylendiaminverbindungen, wobei das Rohmaterial A eine als Formel I gezeigte Struktur aufweist, wobei R' aus Phenyl oder o-Methylphenyl ausgewählt ist, wodurch N-Phenyl-p-phenylendiamin oder N-o-Methylphenyl-p-phenylendiamin sind, wobei der Rohstoff B Cyclohexanon und/oder o-Methylcyclohexanon ist und der Wasserstoffakzeptor Phenol und/oder o-Kresol ist, das Wasserstoff zur Umwandlung in den Rohstoff B aufnehmen kann, 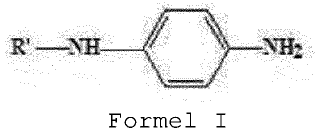 wobei ein Molverhältnis des Rohmaterials A und des Rohmaterials B 20:1≈5:1 beträgt und ein Molverhältnis des Rohmaterials A und des Wasserstoffakzeptors 1:10≈1:2,5 beträgt.

2. Verfahren zur Herstellung nach Anspruch 1, wobei die arylsubstituierten p-Phenylendiaminverbindungen eine Mischung aus N,N'-Diphenyl-p-phenylendiamin, N-Phenyl-N'-methylphenyl-p-phenylendiamin und N,N'-di(Methylphenyl)-p-phenylendiamin sind und das Verfahren zur Herstellung die folgenden Schritte umfasst:
Nehmen des N-Phenyl-p-phenylendiamins als Rohmaterial A, Nehmen des Cyclohexanons und/oder des o-Methylcyclohexanons als Rohmaterial B, Nehmen des Phenols als Wasserstoffakzeptor und Durchführen der Reaktion in Gegenwart des Katalysators, um eine erste Komponente zu erhalten;
Nehmen des N-Phenyl-p-phenylendiamins als Rohmaterial A, Nehmen des Cyclohexanons und/oder des o-Methylcyclohexanons als Rohmaterial B, Nehmen des o-Kresols als Wasserstoffakzeptor und Durchführen der Reaktion in Gegenwart des Katalysators, um eine zweite Komponente zu erhalten oder Nehmen des N-o-Methylphenyl-p-phenylendiamins als Rohmaterial A, Nehmen des Cyclohexanons und/oder des o-Methylcyclohexanons als Rohmaterial B, Nehmen des Phenols als Wasserstoffakzeptor und Durchführen der Reaktion in Gegenwart des Katalysators, um eine zweite Komponente zu erhalten;
Nehmen des N-o-Methylphenyl-p-phenylendiamins als Rohmaterial A, Nehmen des Cyclohexanons und/oder des o-Methylcyclohexanons als Rohmaterial B, Nehmen des o-Kresols als Wasserstoffakzeptor und Durchführen der Reaktion in Gegenwart des Katalysators, um eine dritte Komponente zu erhalten; und
Mischen der ersten Komponente, der zweiten Komponente und der dritten Komponente, um die Mischung aus N, N'-Diphenyl-p-phenylendiamin, N-Phenyl-N'-methylphenyl-p-phenylendiamin und N,N'-di(methylphenyl)-p-phenylendiamin zu erhalten.

3. Verfahren zur Herstellung nach Anspruch 1, wobei die arylsubstituierten p-Phenylendiaminverbindungen eine Mischung aus N,N'-Diphenyl-p-phenylendiamin, N-Phenyl-N'-methylphenyl-p-phenylendiamin und N,N'-di(Methylphenyl)-p-phenylendiamin sind und das Verfahren zur Herstellung die folgenden Schritte umfasst:
Nehmen einer Mischung aus N-Phenyl-p-phenylendiamin und N-o-Methylphenyl-p-phenylendiamin als Rohmaterial A,
Nehmen des Cyclohexanons und/oder des o-Methylcyclohexanons als Rohmaterial B und Nehmen einer Mischung aus Phenol und o-Kresol als Wasserstoffakzeptor und Durchführen der Reaktion in Gegenwart des Katalysators, um die Mischung aus N,N'-Diphenyl-p-phenylendiamin, N-Phenyl-N'-methylphenyl-p-phenylendiamin und N,N'-di-(methylphenyl) p-phenylendiamin zu erhalten.

4. Verfahren zur Herstellung nach einem der Ansprüche 1-3, wobei der Katalysator ein geträgerter Edelmetallkatalysator ist und der Katalysator vorzugsweise ein Pd-C- und/oder Pt-C-geträgerter Edelmetallkatalysator ist.

5. Verfahren zur Herstellung nach Anspruch 4, wobei eine Verwendungsmenge des Katalysators 0,1∼2% Gew.-% des Rohmaterials A beträgt.

6. Verfahren zur Herstellung nach einem der Ansprüche 1-3, wobei das Rohmaterial A und das Rohmaterial B und der Wasserstoffakzeptor für eine Reaktionszeit von 6∼8 Stunden unter einer Temperaturbedingung von 220∼280 °C umgesetzt werden.

7. Verfahren zur Herstellung nach Anspruch 6, wobei nach Beendigung der Reaktion das Verfahren zur Herstellung ferner umfasst: Filtrieren der durch die Reaktion erhaltenen Reaktionsflüssigkeit, um ein Filtrat zu erhalten, und Durchführen einer Destillation unter vermindertem Druck an dem Filtrat, um die arylsubstituierte p-Phenylendiamin-Verbindung zu erhalten.

8. Verfahren zur Herstellung nach Anspruch 6, wobei das Rohmaterial A, das Rohmaterial B und der Wasserstoffakzeptor in einer Stickstoffatmosphäre umgesetzt werden.

## Revendications

1. Procédé de préparation d'un ou plusieurs composé(s) p-phénylènediamine substitué(s) par aryle tel que défini dans une formule structurelle comme suit : 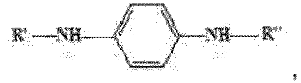 où R" et R' sont choisis indépendamment parmi le phényle ou l'o-méthylphényle ; et
le procédé de préparation comprend : faire réagir une matière première A, une matière première B et un accepteur d'hydrogène en présence d'un catalyseur pour former les composés p-phénylènediamine substitués par aryle, la matière première A ayant une structure représentée par la formule I, où R' est choisi parmi le phényle ou l'o-méthylphényle, étant ainsi du N-phényl p-phénylènediamine ou du N-o-méthylphényl p-phénylènediamine, la matière première B étant du cyclohexanone et/ou de l'o-méthylcyclohexanone et l'accepteur d'hydrogène étant du phénol et/ou de l'o-crésol pouvant accepter l'hydrogène pour la conversion en la matière première B, 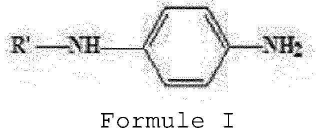 dans laquelle un rapport molaire de la matière première A et de la matière première B est de 20:1∼5:1 et un rapport molaire de la matière première A et de l'accepteur d'hydrogène est de 1:10-1:2,5.

2. Procédé de préparation selon la revendication 1, dans lequel les composés p-phénylènediamine substitués par aryle sont un mélange de N,N'-diphényl p-phénylènediamine, de N-phényl-N'-méthylphényl p-phénylènediamine et de N,N'-di(méthylphényl) p-phénylènediamine, et le procédé de préparation comprend les étapes suivantes :
prendre le N-phényl p-phénylènediamine comme matière première A, prendre le cyclohexanone et/ou l'o-méthylcyclohexanone comme matière première B, prendre le phénol comme accepteur d'hydrogène, et effectuer la réaction en présence du catalyseur pour obtenir un premier composant ;
prendre le N-phényl p-phénylènediamine comme matière première A, prendre le cyclohexanone et/ou le o-méthylcyclohexanone comme matière première B, prendre le o-crésol comme accepteur d'hydrogène et effectuer la réaction en présence du catalyseur pour obtenir un deuxième composant, ou prendre le N-o-méthylphényl p-phénylènediamine comme matière première A, prendre le cyclohexanone et/ou l'o-méthylcyclohexanone comme matière première B, prendre le phénol comme accepteur d'hydrogène, et effectuer la réaction en présence du catalyseur pour obtenir un deuxième composant ;
prendre le N-o-méthylphényl p-phénylènediamine comme matière première A, prendre le cyclohexanone et/ou le o-méthylcyclohexanone comme matière première B, prendre le o-crésol comme accepteur d'hydrogène et effectuer la réaction en présence du catalyseur pour obtenir un troisième composant ; et
mélanger le premier composant, le deuxième composant et le troisième composant pour obtenir le mélange de N,N'-diphényl p-phénylènediamine, de N-phényl-N'-méthylphényl p-phénylènediamine et de N,N'-di(méthylphényl) p-phénylènediamine.

3. Procédé de préparation selon la revendication 1, dans lequel les composés p-phénylènediamine substitués par aryle sont un mélange de N,N'-diphényl p-phénylènediamine, de N-phényl-N'-méthylphényl p-phénylènediamine et de N,N'-di(méthylphényl) p-phénylènediamine, et le procédé de préparation comprend les étapes suivantes :
prendre un mélange de N-phényl p-phénylènediamine et de N-o-méthylphényl p-phénylènediamine comme matière première A, prendre le cyclohexanone et/ou l'o-méthylcyclohexanone comme matière première B, prendre un mélange de phénol et de o-crésol comme accepteur d'hydrogène, et effectuer la réaction en présence du catalyseur pour obtenir le mélange de N,N'-diphényl p-phénylènediamine, de N-phényl-N'-méthylphényl p-phénylènediamine et de N,N'-di(méthylphényl) p-phénylènediamine.

4. Procédé de préparation selon l'une quelconque des revendications 1-3, dans lequel le catalyseur est un catalyseur supporté à métaux nobles, et, de préférence, le catalyseur est un catalyseur supporté à métaux nobles Pd/C et/ou Pt/C.

5. Procédé de préparation selon la revendication 4, dans lequel une quantité d'utilisation du catalyseur est de 0,1 ∼ 2 % en poids de la matière première A.

6. Procédé de préparation selon l'une quelconque des revendications 1-3, dans lequel la matière première A et la matière première B et l'accepteur d'hydrogène sont faits réagir pendant un temps de réaction de 6∼8 heures dans des conditions de température de 220∼280 °C.

7. Procédé de préparation selon la revendication 6, dans lequel, après la réaction terminée, le procédé de préparation comprend de plus : filtrer le liquide de réaction obtenu par la réaction pour obtenir un filtrat, et effectuer une distillation sous pression réduite sur le filtrat pour obtenir le composé p-phénylènediamine substitué par aryle.

8. Procédé de préparation selon la revendication 6, dans lequel la matière première A, la matière première B et l'accepteur d'hydrogène sont faits réagir dans une atmosphère d'azote.
